# EUROPÄISCHE PATENTANMELDUNG

(11) **EP 0 595 211 A1**
(43) Veröffentlichungstag der Anmeldung: **04.05.1994**
(21) Anmeldenummer: 93117118.5
(22) Anmeldetag: 22.10.1993
(51) Int. Cl.: G01N 33/543, G01N 33/569, G01N 33/577, G01N 33/58

(54) **Verfahren zur simultanen Bestimmung von Antigenen und Antikörpern**

(30) Priorität: 27.10.1992 DE 4236189
(71) Anmelder: BOEHRINGER MANNHEIM GMBH, D-68305 Mannheim (DE)
(72) Erfinder: Bayer, Hubert, Dr., D-69469 Weinheim (DE); Faatz, Elke, Dr., D-82396 Pähl (DE); Wiedmann, Michael, dr., D-82377 Penzberg (DE)

(57) **Zusammenfassung**

Zur simultanen Bestimmung eines Antigens eines Erregers und mindestens eines Antikörpers gegen den gleichen Erreger nach dem Prinzip eines heterogenen Immunoassays wird die Probe mit den Rezeptoren R1 bis R4, wobei R1 und R3 mit dem zu bestimmenden Antigen spezifisch bindefähig sind, R2 und R4 mit dem zu bestimmenden Antikörper spezifisch bindefähig sind, R1 und R2 die Bindung an die Festphase vermitteln und R3 und R4 die gleiche Markierung tragen, inkubiert und nach Trennung der festen von der flüssigen Phase die Markierung in einer der beiden Phasen bestimmt.

## Beschreibung

Die Erfindung betrifft ein Verfahren zur simultanen Bestimmung mindestens eines Antigens eines Erregers und mindestens eines Antikörpers gegen den gleichen Erreger nach dem Prinzip eines heterogenen Immunoassays durch Inkubation der Probe mit den Rezeptoren R1 bis R4, wobei R1 und R3 mit dem zu bestimmenden Antigen spezifisch bindefähig sind, R2 und R4 mit dem zu bestimmenden Antikörper spezifisch bindefähig sind, R1 und R2 die Bindung an die Festphase vermitteln, R3 und R4 die gleiche Markierung tragen, Trennung der festen von der flüssigen Phase und Bestimmung der Markierung in einer der beiden Phasen.

Eine Vielzahl klinisch wertvoller Parameter wird mit immunologischen Nachweisverfahren bestimmt. Für die Immunoassays gibt es eine große Auswahl von homogenen und heterogenen Verfahren. Häufig wird ein heterogenes Verfahren nach dem Sandwich-Prinzip oder einer davon abgeleiteten Variante benutzt. Üblicherweise wird die Probelösung mit einem Rezeptor, der mit der zu bestimmenden Substanz bindefähig ist und eine Markierung trägt und einem an die Festphase gebundenen oder einem an die Bindung an die Festphase vermittelnden, mit der zu bestimmenden Substanz spezifisch bindefähigen Rezeptor inkubiert. Dabei bilden sich an die Festphase über den die Bindung an die Festphase vermittelnden Rezeptor gebundene Komplexe von zu bestimmender Substanz und markiertem Rezeptor. Nach der Trennung der flüssigen von der festen Phase kann die Markierung in einer der beiden Phasen nachgewiesen werden.

Zum Nachweis einer Infektion durch einen Erreger werden entweder die spezifisch gegen diesen Erreger gerichteten Antikörper oder bestimmte Antigene dieses Erregers üblicherweise nachgewiesen. Damit eine Infektion möglichst sicher erkannt werden kann, werden separate Bestimmungen der Antikörper und Antigene durchgeführt. Diese Tests haben eine untere Nachweisgrenze. Werden im Test Meßsignale erhalten, die unterhalb dieser Nachweisgrenze liegen, so wird diese Probe als negativ deklariert. Liegen die Meßsignale oberhalb der Nachweisgrenze, so wird die Probe als positiv deklariert. Bei zweifelhaften Proben ist oft eine Nachmessung notwendig. Trotzdem werden einige Proben, die nur geringe Mengen an Antigen oder Antikörper enthalten, durch diese Begrenzung der Nachweisbarkeit der einzelnen zu bestimmenden Substanzen fälschlich als negativ eingestuft. Diese Problematik tritt vor allem bei der Früherkennung von Infektionsparametern auf.

Aufgabe der vorliegenden Erfindung war es, einen Immunoassay zur Bestimmung von Antigenen eines Erregers und Antikörpern gegen diesen Erreger zu entwickeln, der eine verbesserte Differenzierung zwischen negativen und positiven Proben ermöglicht.

Gelöst wird diese Aufgabe durch ein Verfahren zur simultanen Bestimmung mindestens eines Antigens eines Erregers und mindestens eines Antikörpers gegen den gleichen Erreger nach dem Prinzip eines heterogenen Immunoassay durch Inkubation der Probe mit mindestens einem Rezeptor R1, der mit dem zu bestimmenden Antigen, das nicht mit dem Antigen des Erregers identisch ist, gegen das der zu bestimmende Antikörper gerichtet ist, spezifisch bindefähig ist und an eine Festphase gebunden ist oder gebunden werden kann, mindestens einem Rezeptor R2, der mit dem zu bestimmenden Antikörper spezifisch bindefähig ist und an eine Festphase gebunden ist oder gebunden werden kann, mindestens einem Rezeptor R3, der mit dem zu bestimmenden Antigen, das nicht mit dem Antigen des Erregers identisch ist, gegen das der zu bestimmende Antikörper gerichtet ist, spezifisch bindefähig ist und eine Markierung trägt und mindestens einem Rezeptor R4, der mit dem zu bestimmenden Antikörper spezifisch bindefähig ist und die gleiche Markierung wie der Rezeptor R3 trägt, Trennung der festen von der flüssigen Phase und Bestimmung der Markierung in einer der beiden Phasen.

Ein Verfahren zur Bestimmung mehrerer Substanzen, d. h. mehrerer Antikörper oder Antigene oder Antigene eines Erregers in Kombination mit Antikörpern gegen einen anderen Erreger in einem Immunoassay ist in der EP-A-0 379 216 beschrieben. Es werden mehrere Rezeptoren R1 verwendet, die spezifisch mit den zu bestimmenden Substanzen binden. Die Rezeptoren R1 vermitteln die Bindung an die Festphase. Weiterhin können verschiedene Rezeptoren R2 verwendet werden, die mit den verschiedenen zu bestimmenden Substanzen bindefähig sind. Die Rezeptoren R2 tragen entweder verschiedene Markierungen oder aber die gleiche Markierung. Falls verschiedene Markierungen benutzt werden, kann das Verfahren einphasig durchgeführt werden, d. h. die Rezeptoren R2 werden gleichzeitig zugegeben. Falls die gleiche Markierung verwendet wird, muß das Verfahren zweiphasig durchgeführt werden, d. h. die verschiedenen Rezeptoren R2 werden nacheinander zugegeben und die einzelnen Markierungen so nacheinander bestimmt. Eine simultane Bestimmung von Antigenen eines bestimmten Erregers und Antikörper, die gegen diesen bestimmten Erreger gerichtet sind und eine gute Differenzierung zwischen negativen und positiven Proben ermöglicht, wird in der EP-A-0 379 216 nicht offenbart.

Die simultane Bestimmung von Antigenen eines Erregers und Antikörpern, die gegen diese bestimmten Erreger gerichtet sind, wird ermöglicht, da erfindungsgemäß als Rezeptoren R1 und R3 solche Rezeptoren eingesetzt werden, die das zu bestimmende Antigen spezifisch binden, aber nicht gegen ein Antigen gerichtet sind, gegen das der zu bestimmende Antikörper gerichtet ist. Falls Rezeptoren eingesetzt wurden, die ebenfalls das Antigen spezifisch binden, die von dem zu bestimmenden Antikörper gebunden werden, käme es zur gegenseitigen Neutralisation von Antigenen und Antikörpern.

Da es mit dem erfindungsgemäßen Verfahren möglich ist, gleichzeitig Antigene eines Erregers und Antikörper, die gegen diesen Erreger gerichtet sind, in Proben nachzuweisen, summieren sich die Meßsignale, die durch die Antigene und Antikörper hervorgerufen werden. Proben mit einem geringen Gehalt an Antigenen und Antikörpern, die bei einer getrennten Bestimmung der Antigene oder Antikörper grenzwertige Signale, d. h. Signale, die in der Nähe der Nachweisgrenze liegen, liefern und eventuell fälschlich als negativ eingestuft wurden bzw. eine Nachbestimmung erforderten, können mit dem erfindungsgemäßen Verfahren als positiv eingestuft werden. Damit entfällt die zeitaufwendige Nachbestimmung. Weiterhin besteht die Möglichkeit, Patienten nach erfolgter Infektion früher als infiziert zu erkennen und somit die Behandlung früher zu starten. Dies war überraschend, da man davon ausging, daß zu Beginn einer Infektion nur Antigene nachzuweisen sind.

Bei der Auswahl der Rezeptoren R1 bis R4 ist es wichtig, falls eine Früherkennung von Infektionen mit den Verfahren beabsichtigt wird, solche Rezeptoren einzusetzen, die gegen frühe Antigene, d. h. Antigene, die sehr früh nach der Infektion auftreten, bzw. gegen Antikörper, die sehr früh nach der Infektion in hohen Titern in den Proben vorkommen, gerichtet sind. Falls lediglich beabsichtigt wird, mit dem erfindungsgemäßen Verfahren eine verbesserte Differenzierung zwischen negativen und positiven Proben zu ermöglichen und es nicht speziell der Früherkennung von Infektionskrankheiten dient, sondern beispielsweise der Verlaufskontrolle einer Infektionskrankheit, ist es auch möglich, Rezeptoren R1 bis R4 zu verwenden, die gegen Antigene bzw. Antikörper gerichtet sind, die erst später im Verlauf der Infektion auftreten. Die Auswahl der Rezeptoren hängt also davon ab, welches Stadium der Infektionskrankheit bestimmt werden soll. Besonders geeignet ist das erfindungsgemäße Verfahren zur Früherkennung von Infektionskrankheiten.

Das erfindungsgemäße Verfahren wird nach dem Prinzip eines heterogenen Immunoassays durchgeführt. Es wird eine Festphase verwendet, an deren Oberfläche die Rezeptoren R1 und R2 direkt gebunden sind oder gebunden werden können.

Für die Festphase können die an sich bekannten Materialien, wie Kunststoff, Glas, Papierträger, Keramik, Latex, Magnetpartikel, verwendet werden. Die feste Phase kann beispielsweise in Form von Reaktionsröhrchen, Reagenzträgerstreifen oder Kugeln vorliegen. In einer bevorzugten Ausführungsform liegt die Festphase in Form eines Reaktionsröhrchens, dessen Wände zumindest teilweise an der Innenoberfläche mit den Rezeptoren R1 und R2 beschichtet sind bzw. beschichtet werden können, vor. Als Material für das Reaktionsgefäß sind die bekannten Materialien geeignet. Bevorzugt sind Polystyrol, Copolymere des Polystyrols, Polycarbonate, Polyacrylate und Polymethacrylate.

Die Beschichtung der Festphase mit den Rezeptoren R1 und R2 kann entweder direkt oder indirekt erfolgen. Verfahren zum direkten Binden der Rezeptoren an eine Festphase sind dem Fachmann bekannt. Bevorzugt ist eine adsorptive Bindung.

Unter indirekter Bindung ist zu verstehen, daß die Rezeptoren R1 und R2 erst kurz vor oder während der immunologischen Reaktion an die Festphase gebunden werden. An die Festphase ist in diesem Fall ein Partner P1 eines spezifisch bindenden Paares P1/P2 gebunden. Der andere Partner P2 des spezifisch bindenden Paares ist jeweils an die Rezeptoren R1 und R2 gekoppelt.

Die Beschichtung der Festphase mit dem spezifisch bindenden Partner P1 kann entweder direkt, über ein Trägermaterial oder einen Spacer erfolgen. Geeignet ist beispielsweise die Bindung an ein lösliches Protein mit einem Molekulargewicht über 500.000, das dann an der Innenoberfläche des Reaktionsgefäßes adsorbiert wird. Ebenso geeignet ist die Bindung über einen Spacer, der über eine funktionelle Gruppe an der Oberfläche des Reaktionsgefäßes kovalent oder adsorptiv gebunden werden kann. Verfahren und Mittel hierzu sind dem Fachmann bekannt. In einer bevorzugten Ausführungsform wird als feste Phase ein Trägermaterial verwendet, das nach dem in der DE-A-3640412.8 beschriebenen Verfahren hergestellt wurde. In einer weiteren bevorzugten Ausführungsform ist die Festphase ein Reagenzträger, der erhalten wurde, indem ein Faservlies aus einer Cellulose/Synthesefasermischung durch Behandlung mit Perjodat aktiviert wurde und mit dem spezifisch bindenden Partner P1, der vorher sauer behandelt wurde, beladen wurde. Ein Verfahren zur Herstellung derartiger Reagenzträger ist beschrieben in DE-A-3543749.

Als spezifisch bindende Paare P1/P2 sind beispielsweise Antigen/Antikörper, Hapten/Antikörper, Lektin/Kohlenhydrate, Biotin/Anti-Biotin-Antikörper, Biotin/Avidin, Biotin/Streptavidin geeignet. Bevorzugt werden an der Festphase mit Biotin bindefähige Partner immobilisiert, insbesondere Streptavidin oder Avidin.

Das Mengenverhältnis der an die Festphase direkt oder indirekt gebundenen Rezeptoren R1 und R2 kann in weiten Bereichen variiert werden. Das bevorzugte Mengenverhältnis der Rezeptoren R1 zu R2 liegt zwischen 10:1 bis 1:10. Das optimale Mengenverhältnis R1:R2 für jeden speziellen Test kann sehr leicht durch Binden verschiedener Mengen an R1 und R2 an die Festphase ermittelt werden. Bei Verwendung eines spezifisch bindenden Paares P1/P2 zur Bindung der Rezeptoren R1 und R2 an die Festphase entspricht das Mengenverhältnis der gebundenen Rezeptoren R1 und R2 in etwa dem Mischungsverhältnis der Rezeptoren R1 und R2 in der Lösung, die zur Beschichtung benutzt wird, da die Bindungsaffinitäten beider Rezeptoren in etwa gleich sind. Falls eine direkte Bindung der Rezeptoren R1 und R2 durchgeführt wird, kann das Mengenverhältnis der direkt gebundenen Rezeptoren R1 und R2 vom Mischungsverhältnis der Rezeptoren R1:R2 in der Lösung, die zur Beschichtung verwendet wird, abweichen, da die Rezeptoren oft unterschiedlich hohe Affinitäten zur Festphase aufweisen. Falls dies gewünscht wird, kann das exakte Mengenverhältnis der an die Festphase gebundenen Rezeptoren R1 und R2 anschließend nach den dem Fachmann bekannten Methoden, beispielsweise durch Bestimmung der Bindungskapazität, für die zu bestimmenden Antigene und Antikörper ermittelt werden. Entsprechend dem für die Rezeptoren R1:R2 gewählten Mengenverhältnis wird auch das Mengenverhältnis von R3:R4 gewählt. Das Mengenverhältnis der Rezeptoren R3:R4 liegt ebenfalls zwischen 10:1 bis 1:10. Das Mengenverhältnis R3:R4 kann dabei gleich oder ähnlich dem Mengenverhältnis R1:R2 sein, es können aber auch Abweichungen vorkommen. Für jeden einzelnen Test kann das optimale Mengenverhältnis R3:R4 sehr leicht durch Mischen verschiedener Mengen an R3 und R4 ermittelt werden.

Als Rezeptor R1 werden Rezeptoren verwendet, die Bindestellen für das zu bestimmende Antigen besitzen. Das zu bestimmende Antigen muß unterschiedlich zu dem Antigen sein, das von dem zu bestimmenden Antikörper erkannt und gebunden wird. Der Rezeptor R1 muß eine andere antigene Determinante oder ein anderes Epitop des Erregers als der zu bestimmende Antikörper spezifisch erkennen. Es ist notwendig, daß der Rezeptor R1 und der zu bestimmende Antikörper nicht stark mit der selben Bindestelle kreuzreagieren. Bei bekannten Erregern, wie beispielsweise Hepatitis-Viren oder HIV, sind die antigenen Determinanten bekannt. Beispielsweise kann man als Rezeptor R1 zum Nachweis einer Hepatitis-B-Virusinfektion einen Rezeptor gegen das Hepatitis-B-surface-Antigen (HBsAg) einsetzen und gleichzeitig als zu bestimmende Antikörper Anti-Hepatitis-B-core-Antikörper (〈HBc〉Ak) nachweisen. Zum Nachweis einer HIV-Infektion kann beispielsweise ein Rezeptor R1 eingesetzt werden, der das p24-Protein bindet, während anti gp32, gp41 oder gp120 Antikörper nachgewiesen werden. Falls bei einem Erreger die antigene Determinanten nicht bekannt sind, so können durch einen Fachmann anhand bekannter Vorversuche zur Bestimmung der Kreuzreaktivität geeignete Rezeptoren R1 ausgewählt werden, die nicht mit dem zu bestimmenden Antikörper um die selbe Bindestelle konkurrieren.

Als Rezeptoren R1 können auch Gemische aus verschiedenen Rezeptoren verwendet werden, die mit verschiedenen Antigenen des Erregers spezifische bindefähig sind. Beim Nachweis einer HIV-Infektion können beispielsweise Rezeptoren für die antigenen Determinanten p24 und gp32 und/oder gp41 eingesetzt werden. Als zu bestimmender Antikörper wird in diesem Fall beispielsweise ein anti gp120-Antikörper ausgewählt. Im Falle des Nachweises einer Hepatitis-B-Virusinfektion kann z. B. als Rezeptor R1 ein Gemisch aus zwei Rezeptoren für HBsAg und HBcAg eingesetzt werden, während der zu bestimmende Antikörper gegen ein anderes Epitop des HBcAg oder gegen HBeAG gerichtet ist.

Als Rezeptoren R1 können vollständige Antikörper aller Subklassen, die mit dem zu bestimmenden Antigen bindefähig sind, verwendet werden. Statt der vollständigen Antikörper können selbstverständlich auch deren Fragmente, wie Fab-, Fab'- oder F(ab')₂-Fragmente, verwendet werden. Die Antikörper können polyklonal oder monoklonal sein. Da keine Kreuzreaktivität mit den nachzuweisenden Antikörpern auftreten soll, ist es vorteilhaft, wenn hochgereinigte spezifische polyklonale Antiköper verwendet werden. Am geeignetsten ist die Verwendung von monoklonalen Antikörpern.

In einer bevorzugten Ausführungsform des erfindungsgemäßen Verfahrens ist an den Rezeptor R1 ein Partner P2 des spezifisch bindenden Paares P1/P2 gekoppelt. Über dem Partner P2, der mit dem Partner P1 bindet, wird die Immobilisierung an die Festphase bewirkt. Bevorzugt wird als Partner P1 ein Hapten, wie FITC, p-Nitrophenol, Saponin, Digoxin, besonders bevorzugt Biotin verwendet. Verfahren zum Koppeln der Partner P2 an den Rezeptor R2 sind dem Fachmann bekannt.

Als Rezeptor R2 werden die Antigene benutzt, mit denen der oder die zu bestimmenden Antikörper spezifisch binden. Als Antigen können vollständige oder gereinigte Teile von Krankheitserregern, wie Antigene, Protozoen-Antigene oder virale Antigene, verwendet werden. Die Antigene können aus nativem Material, rekombinantem Material oder aus chemisch synthetisierten oder modifizierten Proteinen oder Kohlenhydraten bestehen. So werden beispielsweise beim Nachweis von HIV-Antikörpern als Rezeptor R2 verschiedene antigene Determinanten, wie p24, gp41, gp32 oder gp120, verwendet. Beim Nachweis von Hepatitis-Erkrankungen können die Virus-Antigene HBcAg, HBsAg, HBeAg, HAV etc. verwendet werden. Die Auswahl des Rezeptors R2 hängt, wie oben beschrieben, von dem verwendeten Rezeptor R1 ab.

Ebenso, wie für den Rezeptor R1 beschrieben, kann auch für den Rezeptor R2 ein Gemisch aus verschiedenen Rezeptoren verwendet werden, die mit verschiedenen Antikörpern, die gegen verschiedene antigene Determinanten eines Erregers gerichtet sind, spezifisch bindefähig sind. Beispielsweise kann ein Gemisch aus den Virus-Antigenen gp32, gp41 und gp120 beim Nachweis einer HIV-Infektion Verwendung finden. Der Rezeptor R1 wäre in diesem Fall beispielsweise gegen p24 gerichtet.

Der Rezeptor R2 ist entweder direkt oder indirekt an die Festphase gebunden. In der bevorzugten Ausführungsform des erfindungsgemäßen Verfahrens ist an dem Rezeptor R2 ein Partner P2 des spezifisch bindenden Paares P1/P2 gekoppelt. Als Partner P2 sind an den Rezeptor R2 bei einem bestimmten Test die gleichen Substanzen zu koppeln wie an den Rezeptor R1. Die Auswahl des Partners P2 aus verschiedenen Substanzen sowie die Kopplung erfolgt analog wie beim Rezeptor R1 beschrieben.

Für Rezeptor R3 werden entsprechend dem Rezeptor R1 solche Rezeptoren verwendet, die Bindestellen für das zu bestimmende Antigen besitzen, aber nicht gegen das Antigen gerichtet sind, das der zu bestimmende Antikörper spezifisch bindet. Als Rezeptor R3 können die selben Antikörper verwendet werden, die auch beim Rezeptor R1 Verwendung finden. Die für den Rezeptor R3 verwendeten Antikörper können in einem bestimmten Test abhängig von der durchzuführenden Bestimmung, d. h. der nachzuweisenden Infektion, identisch mit dem für Rezeptor R1 verwendeten Antikörper oder davon verschieden sein. Beispielsweise kann als Rezeptor R1 ein vollständiger Antikörper und als Rezeptor R3 ein Antikörper-Fragment, die beide mit dem selben Antigen spezifisch binden, eingesetzt werden. Die Rezeptoren R1 und R3 müssen das gleiche Antigen spezifisch binden. Sie können dabei aber an verschiedene Epitope des gleichen Antigens binden. Bevorzugt ist, daß die Rezeptoren R1 und R3 nicht um das selbe Epitop des gleichen Antigens konkurrieren. Besonders leicht kann diese Bedingung erfüllt werden, wenn monoklonale Antikörper als R1 und R3 verwendet werden. Wie bei dem Rezeptor R1 kann auch für den Rezeptor R3 ein Gemisch aus verschiedenen Rezeptoren verwendet werden.

Der Rezeptor R3 trägt weiterhin eine Markierung. Zur Markierung sind viele Möglichkeiten bekannt und im erfindungsgemäßen Verfahren geeignet. Als Beispiel können radioaktive Isotope, wie ¹²⁵J, ¹²¹J, ⁵¹Cr, ³⁵S und ³H, Enzyme, wie Peroxidase, β-Galaktosidase oder alkalische Phosphatase, fluoreszierende, chemilumineszierende oder in anderer Weise nachweisbare Substanzen verwendet werden. Neben einer direkten Markierung kann der Rezeptor R3 auch eine indirekte Markierung tragen, indem eine mit dem Rezeptor R3 bindefähige Komponente, die markiert ist, an diesen im Verlauf der Testdurchführung bindet. Beispielsweise kann ein markierter Antikörper, der den Fc-Teil des Rezeptors R3 bindet, verwendet werden. Bevorzugt ist eine Kopplung über eine Hapten-Antikörper-Bindung. In diesem Fall wird an den Rezeptor ein Hapten, wie Digoxin, gekoppelt. An das Hapten bindet ein markierter Anti-Hapten-Antikörper beispielsweise ein Anti-Digoxin-Antikörper-Peroxidase-Konjugat.

Als Rezeptor R4 wird ein Rezeptor eingesetzt, der mit dem zu bestimmenden Antikörper spezifisch bindefähig ist und die gleiche Markierung wie der Rezeptor R3 trägt. Als Markierung sind die gleichen nachweisbaren Substanzen wie bei R3 definiert zu verwenden. Als Rezeptor R4 können die gleichen Antigene benutzt werden, die auch für den Rezeptor R2 eingesetzt werden. Wie bei dem Rezeptor R2 kann für den Rezeptor R4 ein Gemisch von verschiedenen Rezeptoren verwendet werden.

In einer weiteren Verfahrensvariante kann für den Rezeptor ein Antikörper oder ein Fragment davon eingesetzt werden, der den zu bestimmenden Antikörper spezifisch erkennt. Bei dieser Verfahrensvariante existieren zwei Möglichkeiten. Bei der ersten Möglichkeit wird als Rezeptor R4 ein Antikörper verwendet, der nur spezifisch den nachzuweisenden Antikörper erkennt und nicht an die Rezeptoren R1 und R3 bindet. Dies kann beispielsweise erreicht werden, indem als Rezeptor R4 ein Antikörper eingesetzt wird, der spezifisch humane Antikörper erkennt und als Rezeptoren R1 und R3 ausschließlich Antikörper verwendet werden, die nicht humanen Ursprungs sind, beispielsweise Maus-Antikörper.

Bei der zweiten Verfahrensvariante wird als Rezeptor R4 ein Antikörper verwendet, der sowohl spezifisch den nachzuweisenden Antikörper erkennt als auch den Rezeptor R3, nicht jedoch den Rezeptor R1. Beispielsweise kann als Rezeptor R4 ein Antikörper, bevorzugt ein Fab- oder Fab'-Fragment, verwendet werden, der den Fc-Teil eines Antikörpers spezifisch bindet. Als Rezeptor R1 wird in diesem Fall ein Antikörper-Fragment verwendet, das keinen Fc-Teil mehr enthält. Als Rezeptor R3 muß in diesem Fall ein vollständiger Antikörper eingesetzt werden. Der Rezeptor R3 trägt somit keine direkt an ihn gekoppelte Markierung, sondern erhält die Markierung über die Bindung des Rezeptors R4, der eine Markierung trägt. Der Rezeptor R3 trägt somit eine indirekte Markierung.

Damit der Rezeptor R3 nicht während der Inkubation durch die Bindung des Rezeptors R4 vernetzt wird, wird als Rezeptor R4 bevorzugt ein monovalentes Antikörper-Fragment, beispielsweise ein Fab- oder Fab'-Fragment, eingesetzt. Bevorzugt wird die Probe zunächst mit den Rezeptoren R1, R2 und R3 inkubiert. Nachdem sich die an die Festphase gebundenen Komplexe gebildet haben, wird nach Entfernen der überschüssigen Rezeptoren mit dem Rezeptor R4 inkubiert und anschließend die Markierung bestimmt.

Die Probelösung kann mit allen Rezeptoren gleichzeitig inkubiert werden. Die simultane Inkubation ist bevorzugt, wenn R3 und R4 direkt markiert sind und die Rezeptoren R1 und R2 direkt an die Festphase gebunden sind. Falls die Rezeptoren R1 und R2 indirekt an die Festphase gebunden werden, können zunächst diese beiden Rezeptoren mit der Festphase vorinkubiert werden. Die Probe wird anschließend gemeinsam mit den Rezeptoren R3 und R4 inkubiert. Weitere Verfahrensvarianten sind möglich und dem Fachmann bekannt. Die einfache Verfahrensführung ermöglicht die Bestimmung in Analysenautomaten. Das Verfahren eignet sich auch für die Schnelldiagnose. Durch simultanen Nachweis von Antigenen und Antikörpern eines Infektionsparameters können Blutproben, die diesen Risikoparameter enthalten, sofort ausgeschieden werden, ohne daß eine genauere Diagnostik notwendig wäre. Andererseits kann bei Untersuchungen in einem Schnelltest vorweg geprüft werden, ob Risikofaktoren vorliegen und falls dies der Fall ist, dann eine genauere Diagnose, beispielsweise ein DNA-Nachweis, angeschlossen werden. Es ist somit erfindungsgemäß möglich, sehr schnell festzustellen, ob bestimmte Hinweise auf eine Erkrankung vorhanden sind.

Mit dem erfindungsgemäßen Verfahren gelingt es, simultan mindestens ein Antigen eines Erregers und mindestens einen Antikörper, der mit demselben Erreger bindefähig ist, nachzuweisen. Als Erreger bzw. Infektionsparameter kommen dabei alle Erreger in Frage, bei denen im Blut ein Antigen- und ein Antikörper-Titer nachgewiesen werden kann, vor allem Viren, wie Hepatitis-Viren, HIV, CMV, EBV und andere humane Viren, Prokaryonten, wie eukaryontische Erreger sowie Protozoen.

Ein weiterer Gegenstand der Erfindung ist ein Reagenz zur Bestimmung eines Antigens eines Erregers und mindestens eines Antikörpers gegen denselben Erreger, das eine Festphase, an deren Oberfläche die Rezeptoren R1 und R2 gebunden sind oder über die Wechselwirkung des spezifisch gebundenen Paares P1/P2 gebunden werden können, jeweils mindestens einen Rezeptor R1, R2, R3 und R4, wie eingangs definiert, sowie gegebenenfalls weitere übliche Hilfsmittel zur Durchführung eines heterogenen Immunoassays, wie Puffer, Detergenzien, Entstörsub-stanzen, Stabilisatoren sowie gegebenenfalls Mittel zur Bestimmung der Markierung, beispielsweise Enzymsubstrate, wie ABTS®, enthält.

Die Erfindung wird durch die folgenden Beispiele erläutert.

### Beispiel 1:

### HIV-Ag/Anti-HIV-Test

HIV-Antigene (p24-Antigen) und Anti-HIV-Antikörper (Anti gp32, Anti gp41 und Anti gp120) werden in einem Pseudo-2-Schritt-Immunoassay bestimmt.

### Reagenz 1:

- 40 mmol/l Phosphatpuffer, pH 7,0
- 1 Gew.-% Nonidet® P40

### Reagenz 2:

- 40 mmol/l Phosphatpuffer, pH 7,0
- 0,9 Gew.-% NaCl
- 0,2 Gew.-% Rinderserumalbumin
- jeweils 100 ng/ml eines oder mehrerer biotinylierter HIV-Antigene (gp32, gp41 oder gp120)
- jeweils 50 ng/ml eines oder mehrerer Digoxin-markierter HIV-Antigene (gp32, gp41 oder gp120)
- 150 ng/ml biotinylierte Anti-HIV p24-Antikörper
- 75 ng/ml Digoxin-markierte Anti-HIV p24-Antikörper
- 150 mU/ml Anti-Digoxin-Antikörper-Peroxidase-Konjugat

Folgende Antigene wurden dabei verwendet:
- chemisch synthetische Peptide
- HIV I gp120 (V3-loop) (EP-A-0 311 219)
- HIV II gp41 (Gnann, J.W. et al., Science 237, 1987, 1346)
- HIV III gp32 (EP-A-0379 216)

Als Antikörper wurden gereinigte humane polyklonale Anti-p24-Antikörper (Fa. Innogenetics) verwendet.

Die Antigene und Antikörper wurden, wie von Leary et al., PNAS, 80 (1983), 4045, beschrieben, mit Biotin oder Digoxin markiert.

Der Test wurde in einem Streptavidin-Thermo-RSA-Polystyroltube, wie in EP-A-0 379 216 beschrieben, durchgeführt.

100 µl Humanserum oder -plasma wurden im Streptavidin-Therma-RSA-Polystyroltube mit 200 µl Reagenz 1 60 Minuten bei 37 °C inkubiert. Anschließend wurden 700 µl Reagenz 2 zugegeben und 120 Minuten bei 37 °C inkubiert. Danach wurde dreimal mit Leitungswasser gewaschen und zur Nachweisreaktion 1 ml ABTS® Substratlösung zugegeben. Nach 60 Minuten Inkubation bei 37 °C wurde die Extinktion bei 422 nm photometrisch gemessen.

Der Anti-HIV-Test wurde unter Verwendung der einzelnen HIV-Antigene und in Kombination aller HIV-Antigene durchgeführt. Dabei zeigte sich, daß die Testführung mit Streptavidin-Thermo-RSA-Konjugat-Polystyroltubes für die simultane Bestimmung von mehreren einzelnen Antikörpern oder einer Antikörper-Population und Antigenen geeignet ist, gleichgültig, ob die Antikörper gegen den selben Virus oder mehrere Viren bzw. deren Antigene gerichtet sind und ob die nachgewiesenen Antigene einem oder mehreren Virusstämmen angehören (Tabelle 1).

**Tabelle 1**

| HIV-Antigene und Antikörper | Negative Kontrolle | Humanserum-Proben | | | | |
|---|---|---|---|---|---|---|
| | | HIV I Ak + Ag | HIV I Ak + Ag | HIV I Ak + Ag | HIV I Ak + Ag | HIV II Ak + Ag |
| | | (Extinktion in mE) | | | | |
| gp 120 (Pep) | 52 | 2161 | 5611 | 3861 | 94 | 63 |
| gp 41 (Pep) | 48 | 2555 | 5735 | 2872 | 89 | 72 |
| gp 32 (Pep) | 44 | 54 | 247 | 64 | 41 | 1389 |
| 〈p24〉 Ak | 56 | 367 | 427 | 76 | 512 | 1597 |
| Peptide + 〈p24〉Ak | 62 | 4123 | 6895 | 5678 | 643 | 2763 |

## Patentansprüche

1. Verfahren zur simultanen Bestimmung mindestens eines Antigens eines Erregers und mindestens eines Antikörpers gegen den gleichen Erreger nach dem Prinzip eines heterogenen Immunoassays durch Inkubation der Probe mit
(I) mindestens einem Rezeptor R1, der mit dem zu bestimmenden Antigen, das nicht mit dem Antigen des Erregers identisch ist, spezifisch bindefähig ist und an eine Festphase gebunden ist oder gebunden werden kann,
(II) mindestens einem Rezeptor R2, der mit dem zu bestimmenden Antikörper spezifisch bindefähig ist und an eine Festphase gebunden ist oder gebunden werden kann,
(III) mindestens einem Rezeptor R3, der mit dem zu bestimmenden Antigen, das nicht mit dem Antigen des Erregers identisch ist, gegen das der zu bestimmende Antikörper gerichtet ist, spezifisch bindefähig ist und eine Markierung trägt,
(IV) mindestens einem Rezeptor R4, der mit dem zu bestimmenden Antikörper spezifisch bindefähig ist und die gleiche Markierung wie der Rezeptor R3 trägt,
Trennung der festen von der flüssigen Phase und Bestimmung der Markierung in einer der beiden Phasen.

2. Verfahren gemäß Anspruch 1, dadurch gekennzeichnet, daß die Rezeptoren R1 und R3 jeweils Gemische aus verschiedenen Rezeptoren sind, die mit verschiedenen zu bestimmenden Antigenen des Erregers spezifisch bindefähig sind.

3. Verfahren gemäß einem der Ansprüche 1 oder 2, dadurch gekennzeichnet, daß die Rezeptoren R1 und R3 monoklonale oder polyklonale Antikörper sind.

4. Verfahren gemäß Anspruch 3, dadurch gekennzeichnet, daß die Rezeptoren R1 und R3 monoklonale oder polyklonale Anti-HIV p24-Antikörper sind.

5. Verfahren gemäß einem der Ansprüche 1 bis 4, dadurch gekennzeichnet, daß die Rezeptoren R2 und R4 jeweils Gemische aus verschiedenen Rezeptoren sind, die mit den zu bestimmenden Antikörpern spezifisch bindefähig sind.

6. Verfahren gemäß einem der Ansprüche 1 bis 5, dadurch gekennzeichnet, daß die Rezeptoren R2 und R4 vollständige oder gereinigte Teile von Erregern wie bakterielle Antigene, Protozoen-Antigene oder virale Antigene sind.

7. Verfahren gemäß Anspruch 6, dadurch gekennzeichnet, daß die viralen Antigene synthetisierte Peptide sind.

8. Verfahren gemäß Anspruch 7, dadurch gekennzeichnet, daß die synthetisierten Peptide die HIV I-Peptide gp120, gp41 und gp32 sind.

9. Verfahren gemäß einem der Ansprüche 1 bis 4, dadurch gekennzeichnet, daß der Rezeptor R4 ein Antikörper ist, der den oder die zu bestimmenden Antikörper spezifisch bindet.

10. Verfahren gemäß der Ansprüche 1 bis 9, dadurch gekennzeichnet, daß an die Rezeptoren R1 und R2 ein Partner P2 eines spezifisch bindenden Paares P1/P2 gekoppelt ist, der Partner P1 des spezifisch bindenden Paares an die Festphase gebunden ist und durch die spezifische Bindung von P1 an P2 die Rezeptoren R1 und R2 an die Festphase gebunden werden.

11. Verfahren gemäß einem der Ansprüche 1 bis 10, dadurch gekennzeichnet, daß die Rezeptoren R3 und R4 eine indirekte Markierung tragen.

12. Verfahren gemäß Anspruch 11, dadurch gekennzeichnet, daß die indirekte Markierung über eine Hapten-Antikörper-Bindung zustande kommt, wobei das Hapten an die Rezeptoren R3 und R4 gekoppelt ist und der haptenspezifische Antikörper eine Markierung trägt.

13. Verfahren gemäß Anspruch 12, dadurch gekennzeichnet, daß als Hapten Digoxin und als markierter Antikörper ein Anti-Digoxin-Antikörper-Peroxidase-Konjugat verwendet werden.

14. Reagenz zur Bestimmung mindestens eines Antigens eines Erregers und mindestens eines Antikörpers gegen den selben Erreger umfassend,
(1) eine Festphase, an deren Oberfläche die Rezeptoren R1 und R2 gebunden sind oder gebunden werden können,
(II) mindestens einen Rezeptor R1, der mit dem zu bestimmenden Antigen, das nicht mit dem Antigen des Erregers identisch ist, spezifisch bindefähig ist und an die Festphase gebunden ist oder gebunden werden kann,
(III) mindestens einen Rezeptor R2, der mit dem zu bestimmenden Antikörper spezifisch bindefähig ist und an die Festphase gebunden ist oder gebunden werden kann,
(IV) mindestens einen Rezeptor R3, der mit dem zu bestimmenden Antigen, das nicht mit dem Antigen des Erregers identisch ist, gegen das der zu bestimmende Antikörper gerichtet ist, spezifisch bindefähig ist und eine Markierung trägt,
(V) mindestens einen Rezeptor R4, der mit dem zu bestimmenden Antikörper spezifisch bindefähig ist und die gleiche Markierung wie der Rezeptor R3 trägt sowie
(VI) gegebenenfalls weitere übliche Hilfsmittel.
